# EUROPEAN PATENT SPECIFICATION

(11) **EP 1 852 143 B1**
(45) Date of publication and mention of the grant of the patent: **10.04.2013**
(21) Application number: 06113442.5
(22) Date of filing: 03.05.2006
(51) Int. Cl.: A61N 1/36, A61N 1/05

(54) **Electrode for implantation in a living organ**
Elektrode für Implantation in einem lebenden Organ
Électrode pour l'implantation dans un organe vivant

(43) Date of publication of application: 07.11.2007
(73) Proprietor: Medtronic, Inc., Minneapolis, MN 55432 (US)
(72) Inventor: Lönroth, Hans, 429 33 Kullavik (SE); Abrahamsson, Hasse, 430 94 Bohus Björkö (SE)
(74) Representative: Lind, Urban Arvid Oskar

(56) References cited:
- WO-A-99/44675
- US-A1- 2004 162 595
- US-A1- 2004 243 195
- US-A1- 2005 021 101

## Description

### Field of the invention

The present invention relates to an electrode intended to be implanted into a living organ, such as the stomach, for emission or reception of electrical signals, and to be connected to an electric device, such as e. g. a pacemaker.

### Background of the invention

The states of ill-health that result in disorder of the motor functions of the stomach and the intestinal canal still are not fully understood, although they may seriously handicap afflicted patient groups. A condition known as gastropares (i. e. cease of motor function in the stomach) may afflict for instance patients suffering from highly advanced diabetes. This condition may be very difficult to treat. In order to decrease symptoms and improve motor functions of the intestinal canal under these circumstances and thus to accelerate the bowel movements, the pacemaker technique can be used.

The pacemaker technique is well-known for mechanical-electrical control of the nerves of a living organ. The technique involves implantation of at least one electrode in the organ concerned. Insulated lines connect the electrode to a pacemaker which is implanted underneath the patient's skin. The pacemaker generates electric impulses controlling the functions of the organ via lines and electrodes.

When the pacemaker technique is used to control symptoms and improve the motor functions of the stomach and the intestinal canal the electrodes are implanted in the wall of the stomach.

The electrodes supplied for this purpose are implanted by being sewn into the stomach wall, which is done by open abdominal surgery or with the aid of the laparoscopic surgery technique. Both implantation methods are serious operations requiring that the patient be anaesthetized.

Implantation of electrodes into a stomach wall poses special problems since the organ wall yields as the electrodes are being implanted. In addition, the risks of inadvertent puncture of the stomach wall are not negligible.

An alternative solution is provided in US 2004/0243195, disclosing a control unit to be arranged inside the stomach, and connected with an anchoring electrode which is introduced through the mouth, and which perforates the stomach wall from the inside. However, even if this procedure does not require open surgery, it is still a rather complicated procedure, and e.g. involves puncturing of the stomach wall.

For the reasons stated there is a need for an electrode that may be implanted with the aid of a less precarious and more simple operation method that may be performed under local anaesthetic conditions. Not only would such an implantation method reduce the costs with respect to the operation as such and to the hospitalization of the patient, but it would also decrease patient discomfort.

Further, it has been empirically established that some patients do not react positively on the pacemaker treatment, and on such patient these relatively complicated, cumbersome and expensive operations are wasted. It is estimated that as much as 20-25% of all patients belong to this non-responsive group. Therefore, it would be advantageous if the patients could be tested before the operation, in order to establish whether they are responsive to the treatment or not, whereby superfluous operations could be saved.

WO 99/44675 discloses an electrode for use e.g. in the stomach wall, and for similar purposes as discussed above. This electrode comprises an electrode head being formed by a coil-shaped contact wire, which wire forms the electrode area. The coil-shaped contact wire can be maintained in a compressed condition during insertion, and then be allowed to expand after insertion, in order to maintain the electrode in its place.

However, a problem with this known electrode is that it is relatively complicated and expensive to produce. Further, with this known electrode it is difficult to find a working compromise between the need to maintain the electrode in its place after implantation, and the need to facilitate insertion and removal.

Still further, in order to make insertion of the electrode possible, the connection cable need to be relatively rigid and stiff, which is a great disadvantage in the use situation, since it incurs a risk that the electrode may be inadvertently removed, pain may be incurred to the patient, etc.

When used in the stomach wall, another disadvantage with the known electrode is that there is a great risk that the electrode contact area comes into contact with the sensitive peritoneum, which may cause severe pain to the patient, which is not only painful and inconvenient for the patient, but may also ruin any diagnostic measurement results.

There is therefore still a need for an improved gastrointestinal electrode.

### Summary of the invention

It is therefore an object of the present invention to provide a new and improved gastrointestinal electrode, which alleviates the above-related drawbacks of the prior art.

This object is achieved with a gastrointestinal electrode according to the appended claims.

According to a first aspect of the invention there is provided a gastrointestinal electrode device comprising an electrode head intended to be implanted in a stomach wall for emission or reception of electrical signals and a flexible cable with an outer isolation layer, said flexible cable being connected to said electrode head for forwarding the electric signals to an external side of said electrode device; wherein said electrode head comprises flexible expandable retention means and at least one electrode contact area arranged to cover at least a part of the outer electrode head area; and wherein the expandable retention means are automatically expandable from an condition of insertion into an expanded condition for opposing retraction of the electrode via the aperture of insertion, thus making the electrode attachable to the living organ. Further, the electrode head comprises an abutment area; and the electrode device comprises a relatively rigid introduction part, arranged to engage with said abutment area of the electrode head for percutanous introduction of the catheter into the stomach wall via an aperture, wherein the introduction part is releasable from the electrode head after insertion, whereby the electrode head remains implanted in the stomach wall.

The electrode is insertable via an aperture, such as e. g. a cannula aperture, and the electrode is expandable into an expanded condition in which the extension of the electrode perpendicularly to the direction of introduction exceeds its extension in the condition of insertion, whereby in its expanded condition said electrode opposes its retraction via the aperture of insertion, thus making the electrode attachable to the stomach wall. Further, the electrode is arranged to expand automatically, i. e. it expands by itself and assumes its expanded condition as soon as it leaves the cannula. An electrode in accordance with the invention thus may be implanted in a stomach wall in a minor and simple surgical operation that may be performed quickly and does not require complete anaesthetization of the patient. The electrode preferably is positioned in the connective tissue between the mucous membrane of the stomach and the muscle layer of the stomach wall. Implantation of electrodes in a wall of a stomach can be performed under assistance by a gastroscopical technique. Hereby, inflation of the organ is performed to expand the organ wall, thus solving the problem connected with the wall yielding as the aperture is to be formed therein, and viewing of the wall from within is rendered possible while the aperture is being formed, thus ensuring that the organ wall is not penetrated inadvertently. Physiological saline solution may advantageously be injected through the aperture before the electrode is inserted. If a cannula is used to form the aperture, the saline solution preferably is injected via the cannula. Preferably, two or more electrodes are implanted, separated by a separation distance ranging between 0.8 and 8 cm, for measuring natural electric signals within the stomach wall (gastric EMG), or for providing electric pacemaker signals to the stomach wall. This treatment (gastric pacemaker stimulation) with the device implanted under abdominal surgery is a new important way of treatment and the main part of the patients respond to GES. However, approximately one fifth of the patients are non-responders. Thus, the electrodes can e.g. be used for gastric electrical stimulation (GES) to treat nausea and vomiting in patients with gastric dysfunction, and/or for recording of the gastric electrical rhythm to study the underlying pathophysiology in patients with severe symptoms and suspicion of motor abnormality. Thus, the new electrodes of the present invention may also be used as a diagnostic pre-study before using other, conventional pacemaker techniques. For a diagnosis to be made, it is desirable that the function of the organ concerned be observed for some length of time. For this reason, the electrodes are connected to a measurement instrument which may be located externally of the patient's body during the observation period.

The relatively rigid introduction part, arranged to engage with said abutment area of the electrode head, makes it possible to introduce the electrode head in an easy, convenient and controllable way into the submucosa, even though a very thin and/or flexible cable. Such a flexible cable is very advantageous, since it allows the electrode to be introduced in the patient for a long duration, such as several weeks, or even months or years, without falling out or causing pain to the patient. This also significantly reduces the risk of infection.

Practical in vivo tests on pigs and humans have shown that the electrode design according to the present invention works very well.

Preferably, the expandable retention means comprises at least one flexible retention wing. The flexible retention wings preferably each has a width that is equal to or less than the diameter of the electrode head. Further, the radial length of each wing is preferably in the range of 1-3 diameters of the electrode head. Preferably, the expandable retention means comprises at least two flexible retention wings, and preferably arranged equidistantly separated in a circumferential direction around the electrode. Hereby, at least one flexible retention wing can be arranged to substantially prevent unintentional release of the electrode when implanted, but allow intentional removal by application of a pulling force to the flexible cable. Thus, the wings are preferably large enough to be held in place under the muscle during ordinary use, but small enough not to get firmly rooted.

Further, the electrode head preferably comprises a rounded tip portion, said rounded tip portion not being a part of the electrode contact area. Due to this rounded distal tip, there is a minimal risk for perforation and penetration through the stomach wall. In the practical tests, no perforations have been experienced.

In the above-described gastrointestinal electrode, the electrode contact area may be arranged between the flexible expandable retention means and a connection between the flexible cable and the electrode head. Hereby, the contact area becomes large enough to provide the necessary electrical contact within the muscle layer, but small enough to greatly alleviate the risk of stimulation of the sensitive peritoneum. For example, the connector part has a diameter of about 1-2 mm, and preferably around 1.2 mm, and a length of about 1-4 mm, and preferably about 2.5-3.0 mm.

In one alternative embodiment, the introduction part can comprise a tube-like part arranged to be arranged outside the flexible cable, wherein said abutment area is formed as protruding shoulder area on said electrode head. Alternatively, the introduction part can comprise a thin stick, arranged to be arranged inside the flexible cable, wherein said abutment area is formed as a central recession or abutment stop in the electrode head. In this latter alternative, the electrode device can overall be made simpler and thinner.

It is also possible to provide two or more separate electrode contact areas on said electrode head, wherein said separate electrode contact areas are connected to separate signal lines in said flexible cable. For example, said separate electrode contact areas can be provided as separate circumferential rings around the electrode head. For example, said electrode contact areas can be arranged on 1-4 mm distance, and preferably about 3 mm. The two or more separate electrode contact areas enables bipolar stimulation or registration by the use of one single electrode.

These and other aspects of the invention will be apparent from and elicidated with reference to the embodiments described hereinafter.

### Brief description of the drawings

For exemplifying purposes, the invention will be described in closer detail in the following with reference to embodiments thereof illustrated in the attached drawings, wherein:
Fig 1 is a schematic overview of the distal end of a gastrointestinal electrode device according to one embodiment of the invention, where said electrode device is arranged in a cannula;
Fig 2a and 2b are schematic overviews of the distal end of the gastrointestinal electrode device of fig 1 without the cannula;
Fig 3a - d are schematic overview illustrating various steps in the operation of inserting the electrode device of fig 1 and 2.
Fig 4 is a schematic overview of a gastrointestinal electrode device in accordance with a second embodiment of the invention; and
Fig 5 is a schematic overview of a gastrointestinal electrode device in accordance with a third embodiment of the invention.

### Description of preferred embodiments

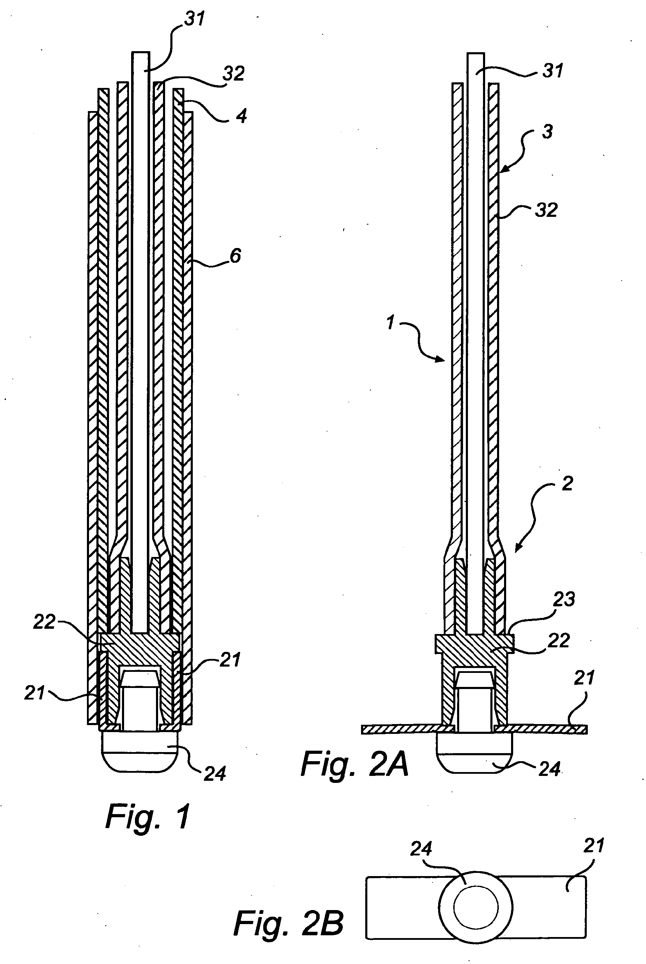

In a first embodiment of the gastrointestinal electrode device according to the invention, is illustrated in fig 1-3. The gastrointestinal electrode device 1 comprises an electrode head 2 intended to be implanted in a stomach wall for emission or reception of electrical signals and a flexible cable 3 with an inner conductor 31 and an outer isolation layer 32. The flexible cable is connected to the electrode head for forwarding the electric signals to electronic equipment (not shown) on the external side, such as a pacemaker or measurement unit. The flexible cable is very thin and/or flexible, so as to be easily bent and folded.

The electrode head 2 comprises flexible expandable retention wings 21, arranged to automatically expand from an condition of insertion, as illustrated in fig 1, into an expanded condition for opposing retraction of the electrode via the aperture of insertion, as illustrated in fig 2, thus making the electrode attachable to the living organ. The flexible retention wings preferably each has a width that is equal to or less than the diameter of the electrode head. Further, the radial length of each wing is preferably in the range of 1-3 diameters of the electrode head. In the illustrated embodiment, two flexible retention wings 21 are arranged equidistantly separated in a circumferential direction around the electrode. Hereby, the at least one flexible retention wing can be arranged to substantially prevent unintentional release of the electrode when implanted, but allow intentional removal by application of a pulling force to the flexible cable. Thus, the wings are preferably large enough to be held in place under the muscle during ordinary use, but small enough not to get firmly rooted.

Further, an electrode contact area 22 is arranged to cover at least a part of the outer electrode head area. This contact area is preferably made of metal, and connected to the connector 31 of the flexible cable.

The electrode contact area 22 is consequently arranged between the flexible expandable wings 21 and the connection between the flexible cable 3 and the electrode head 2. Hereby, the contact area becomes large enough to provide the necessary electrical contact within the muscle layer, but small enough to greatly alleviate the risk of stimulation of the sensitive peritoneum. For example, the connector part has a diameter of about 1-2 mm, and preferably around 1.2 mm, and a length of about 1-4 mm, and preferably about 2.5-3.0 mm.

Further, the electrode head preferably comprises a rounded distal tip portion 24 of an isolation material. Hereby, the rounded tip portion is not a part of the electrode contact area. Due to this rounded distal tip, there is a minimal risk for perforation and penetration through the stomach wall.

Also, the electrode head comprises an abutment area 23. Hereby, a relatively rigid introduction part 4 can be arranged to engage with the abutment area 23 of the electrode head for percutanous introduction of the catheter into a stomach wall into a stomach wall via an aperture. The introduction part is releasable from the electrode head after insertion, whereby the electrode head remains implanted in the stomach wall. In this embodiment, the introduction part is of a tube-like form, for arrangement externally on the flexible cable, and the abutment areas 23 are a radially protruding part of the electrode head, such as a small flange or the like.

Fig. 2 illustrates the electrode 1 in an expanded condition, where the cannula 6 and introduction part 4 have been removed. In this condition, the extension of the electrode 1 perpendicularly to the direction of introduction exceeds its extension in the condition of insertion, a feature which prevents the electrode 1 from being pulled outwardly via the insertion aperture.

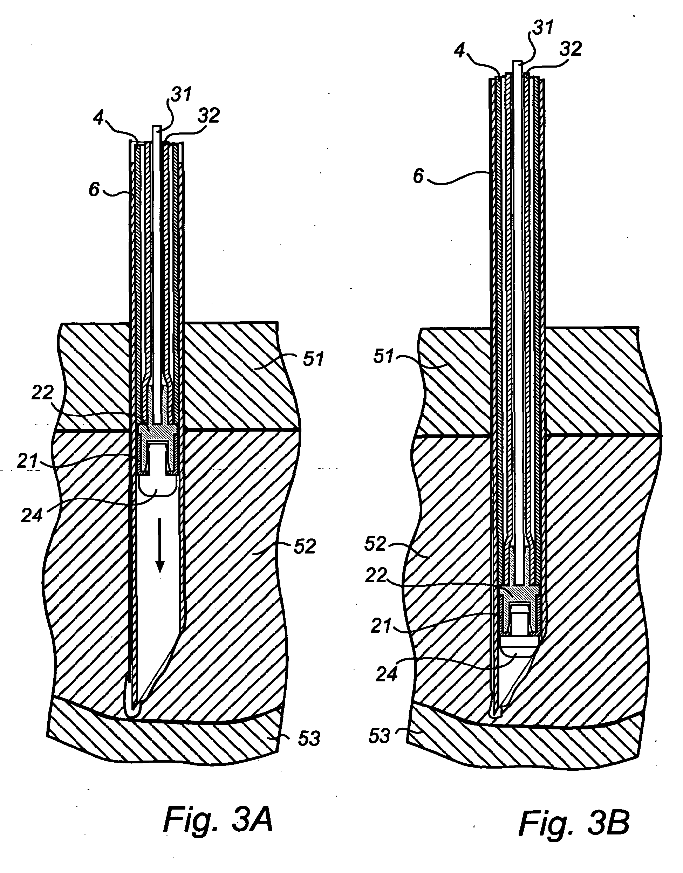

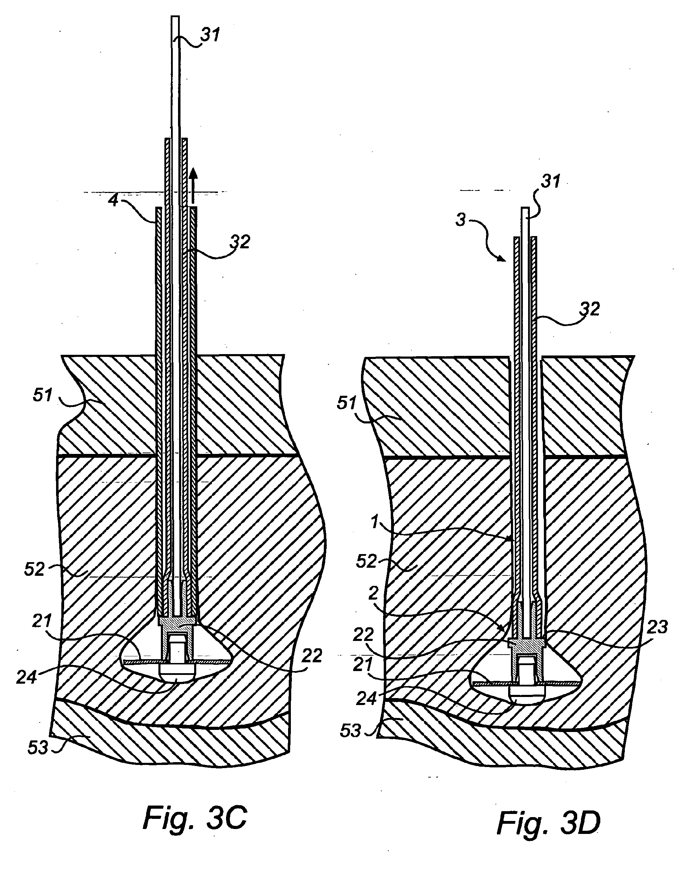

An operation method for insertion of the electrode device is schematically illustrated in figs 3A - 3D.

The stomach wall has a porous connective tissue layer 52 sandwiched between the mucosa 53 of the stomach and a muscular layer 51. In the implantation of an electrode 1 into the stomach wall the stomach may be inflated and viewed from within by means of a gastroscopic instrument.

A cannula 6 is made to penetrate the abdominal wall and the stomach wall 3 to be positioned in the connective tissue layer 52. The cannula is in position in the connective tissue layer 52 when the mucosa 53, as determined by the gastroscopic instrument, is seen to cave inwards under the pressure from the cannula. Physiological saline solution may then be injected through the cannula, thus removing from the cannula any tissue remaining therein and creating a cavity in the connective tissue 52. An electrode 1 of an automatically expanding type is introduced into the cannula, whereby the wings assumes a contracted insertion condition, and further into the cavity created in the connective tissue layer 52. The introduction part 4 is used to stabilize the electrode device during the insertion through the cannula.

As the electrode 1 leaves the cannula, the wings expands automatically into the expanded condition, and in doing so attaches to the stomach wall. The cannula and introduction part are then removed.

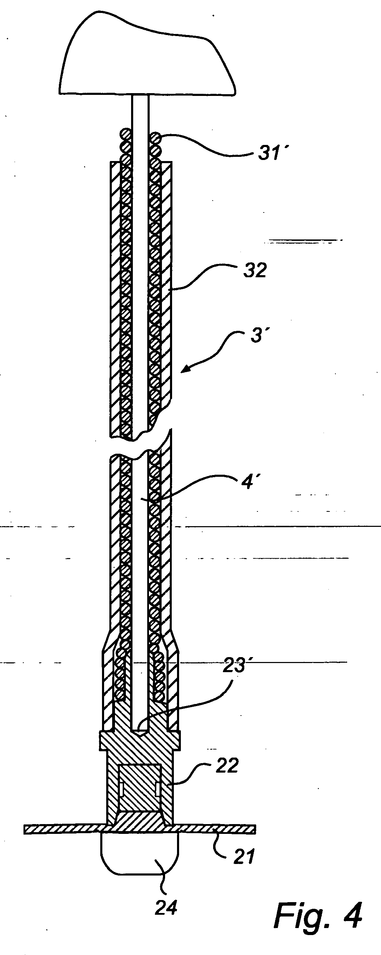

In an alternative embodiment, illustrated in fig 4, the flexible cable 3' is made hollow, where the conductor 31' is arranged around a hollow interior. In the bottom of the hollow interior, an internal abutment area 23' is arranged, as a recess or abutment stop in the electrode head. In this case, the introduction part 4' can comprise a thin stick, arranged to be arranged inside the flexible cable. In this embodiment, the function is essentially the same as in the first described embodiment, but the electrode device can overall be made simpler and thinner. Apart from the above-discussed differences, the second embodiment is essentially the same in function and structure as the above-discussed first embodiment.

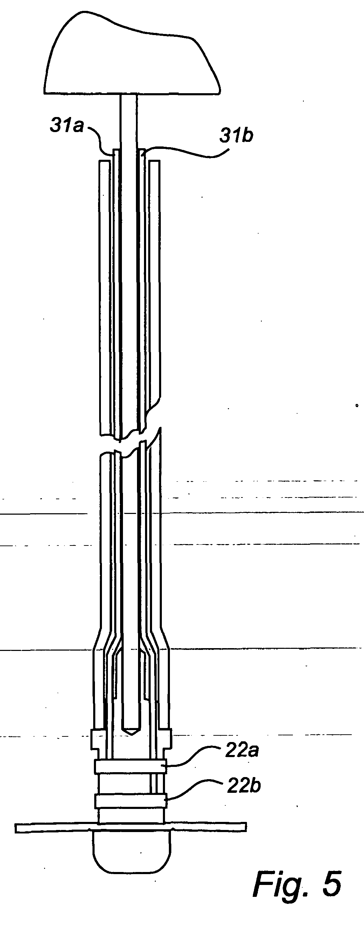

In still another embodiment, illustrated in fig 5, two separate electrode contact areas 22a and 22b are provided on the electrode head. These separate electrode contact areas 22a and 22b are connected to separate conductors 31a and 31b, respectively, in the flexible cable. Here, the separate electrode contact areas are provided as separate circumferential rings around the electrode head. For example, said electrode contact areas can be arranged on 2-4 mm distance from each other, and preferably about 3 mm. The two or more separate electrode contact areas enables bipolar stimulation or registration by the use of one single electrode. Apart from the above-discussed differences, the third embodiment is essentially the same in function and structure as the above-discussed second embodiment. However, it will be appreciated by someone skilled in the art, that two or more separate electrode contact areas may also be provided in the first discussed embodiment.

Practical in vivo tests on pigs and humans have shown that the electrode design according to the present invention works very well.

The measurement in pigs and humans has shown that recording of the gastric electrical rhythm can be made with the new type of electrodes for a long duration of times, typically several days or weeks, providing precise measurement data, and without infections or the like.

In patients, the new electrode device has been used for gastric electrical stimulation to treat nausea and vomiting in patients with gastric dysfunction. This treatment (gastric pacemaker stimulation) with the device implanted under abdominal surgery is a new important way of treatment and the main part of the patients respond to GES. However, approximately one fifth of the patients are found to be non-responders, for which a pacemaker treatment would have essentially no notable beneficial effect. Practical experiments with 15 patients indicates that the percutaneous electrodes are working well in their present form. The stimulations have been performed for a duration of 1-6 weeks, and in a so-called double-blind scheme, where active signals are only provided during certain time periods, whereas during other time periods the electrodes are idle.

In these tests on humans, reliable and adequate diagnostic data in respect of the response to gastric pacemaker stimulation has been provided for all the patients. Specifier the weekly vomiting and nausea frequency of the patients has been studied. Further, no negative side-effects such as infections, pain or other inconveniences has been experienced by the patients.

Specific embodiments of the invention have now been described. However, several alternatives are possible, as would be apparent for someone skilled in the art. For example, it is possible to use other types of abutment areas and/or introducing parts. Still further, a two or more electrode contact areas can be used in all the different embodiments. Also, the arrangement of the contact areas need not be circumferential rings, but sector areas or the like are also feasible.

Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims.

## Claims

1. A gastrointestinal electrode device (1) comprising an electrode head (2) intended to be implanted in a stomach wall for emission or reception of electrical signals and a flexible cable (3; 3') with an outer isolation layer (32), said flexible cable (3; 3') being connected to said electrode head (2) for forwarding the electric signals to an external side of said electrode device (1);
wherein said electrode head (2) comprises flexible expandable retention means and at least one electrode contact area (22) arranged to cover at least a part of the outer electrode head area; and
wherein the expandable retention means are automatically expandable from an a condition of insertion into an expanded condition for opposing retraction of the electrode via the aperture of insertion, thus making the electrode attachable to the stomach wall;
wherein the electrode head (2) further comprises an abutment area (23; 23'); and the electrode device further comprises a relatively rigid introduction part (4; 4'), arranged to engage with said abutment area (23; 23') of the electrode head (2) for percutanous introduction of the catheter into the stomach wall via an aperture, wherein the introduction part (4; 4') is releasable from the electrode head (2) after insertion, whereby the electrode head (2) remains implanted in the stomach wall; and **characterized**
**in that** the expandable retention means comprises at least one flexible retention wing (21);
wherein the electrode head is dimensioned to be fully contained within the stomach wall.

2. The gastrointestinal electrode of claim 1, wherein the at least one flexible retention wing (21) each has a width that is equal to or less than the diameter of the electrode head (2).

3. The gastrointestinal electrode of claim 2, wherein the expandable retention means comprises at least two flexible retention wings (21), preferably arranged equidistantly separated in a circumferential direction around the electrode.

4. The gastrointestinal electrode of any one of the preceding claims, wherein the electrode head (2) further comprises a rounded tip portion (24), said rounded tip portion not being a part of the electrode contact area (22).

5. The gastrointestinal electrode of any one of the preceding claims, wherein the electrode contact area (22) is arranged between the flexible expandable retention means (21) and a connection between the flexible cable (3) and the electrode head (2).

6. The gastrointestinal electrode of any one of the preceding claims, wherein the introduction part (4) comprises a tube-like part arranged to be arranged outside the flexible cable (3), wherein said abutment area (23) is formed as protruding shoulder area on said electrode head (2).

7. The gastrointestinal electrode of any one of the claims 1-5, wherein the introduction part (4') comprises a thin stick, arranged to be arranged inside the flexible cable (3'), wherein said abutment area (23') is formed as a central recession or abutment stop in the electrode head (2).

8. The gastrointestinal electrode of any one of the preceding claims, wherein it is designed for control and/or observation of the motor function in the stomach.

9. The gastrointestinal electrode of any one of the preceding claims, wherein two separate electrode contact areas are provided on said electrode head (2), wherein said separate electrode contact areas (22a, 22b) are connected to separate signal lines in said flexible cable (3; 3').

10. The gastrointestinal electrode of claim 9, wherein the separate electrode contact areas (22a, 22b) are provided as separate circumferential rings around the electrode head (2).

## Patentansprüche

1. Gastrointestinalelektrodenvorrichtung (1), die Folgendes umfasst:
einen Elektrodenkopf (2), der dafür vorgesehen ist, in eine Magenwand eingesetzt zu werden, um elektrische Signale zu senden und zu empfangen; und ein flexibles Kabel (3; 3') mit einer äußeren Isolierschicht (32), wobei das flexible Kabel (3; 3') mit dem Elektrodenkopf (2) verbunden ist, um die elektrischen Signale an eine Außenseite der Elektrodenvorrichtung (1) weiterzuleiten,
wobei der Elektrodenkopf (2) flexible verlängerbare Haltemittel und mindestens eine Elektrodenkontaktfläche (22) umfasst, die dafür konfiguriert ist, mindestens einen Teil der äußeren Elektrodenkopffläche zu bedecken, und
wobei die verlängerbaren Haltemittel automatisch aus einem eingesetzten Zustand in einen verlängerten Zustand verlängerbar sind, um einem Zurückziehen der Elektrode über die Einsetzöffnung entgegenzuwirken, wodurch die Elektrode an der Magenwand angebracht werden kann;
wobei der Elektrodenkopf (2) des Weiteren eine Anliegefläche (23; 23') umfasst; und wobei die Elektrodenvorrichtung des Weiteren einen relativ starren Einführungsteil (4; 4') umfasst, der dafür ausgelegt ist, die Anliegefläche (23; 23') des Elektrodenkopfes (2) für ein perkutanes Einführen des Katheters in die Magenwand über eine Öffnung in Eingriff zu nehmen, wobei der Einführungsteil (4; 4') nach dem Einführen von dem Elektrodenkopf (2) abgenommen werden kann, wobei der Elektrodenkopf (2) in der Magenwand implantiert bleibt; und
**dadurch gekennzeichnet, dass**
das verlängerbare Haltemittel mindestens einen flexiblen Halteflügel (21) umfasst;
wobei der Elektrodenkopf dafür bemessen ist, vollständig in der Magenwand aufgenommen zu werden.

2. Gastrointestinalelektrode nach Anspruch 1, wobei der mindestens eine flexible Halteflügel (21) jeweils eine Breite aufweist, die maximal so groß ist wie der Durchmesser des Elektrodenkopfes (2).

3. Gastrointestinalelektrode nach Anspruch 2, wobei das verlängerbare Haltemittel mindestens zwei flexible Halteflügel (21) umfasst, die bevorzugt in gleichem Abstand voneinander getrennt in einer Umfangsrichtung um die Elektrode herum angeordnet sind.

4. Gastrointestinalelektrode nach einem der vorangehenden Ansprüche, wobei der Elektrodenkopf (2) des Weiteren einen gerundeten Spitzenabschnitt (24) umfasst, wobei der gerundete Spitzenabschnitt nicht zu der Elektrodenkontaktfläche (22) gehört

5. Gastrointestinalelektrode nach einem der vorangehenden Ansprüche, wobei die Elektrodenkontaktfläche (22) zwischen dem flexiblen verlängerbaren Haltemittel (21) und einer Verbindung zwischen dem flexiblen Kabel (3) und dem Elektrodenkopf (2) angeordnet ist.

6. Gastrointestinalelektrode nach einem der vorangehenden Ansprüche, wobei der Einführungsteil (4) einen röhrenartigen Teil umfasst, der dafür konfiguriert ist, außerhalb des flexiblen Kabels (3) angeordnet zu werden, wobei die Anliegefläche (23) als ein hervorstehender Schulterbereich an dem Elektrodenkopf (2) ausgebildet ist.

7. Gastrointestinalelektrode nach einem der Ansprüche 1-5, wobei der Einführungsteil (4') einen dünnen Stab umfasst, der dafür konfiguriert ist, im Inneren des flexiblen Kabels (3') angeordnet zu werden, wobei die Anliegefläche (23') als eine mittige Aussparung oder ein Endanschlag in dem Elektrodenkopf (2) ausgebildet ist.

8. Gastrointestinalelektrode nach einem der vorangehenden Ansprüche, die zur Kontrolle und/oder Beobachtung der motorischen Funktion im Magen vorgesehen ist.

9. Gastrointestinalelektrode nach einem der vorangehenden Ansprüche, wobei zwei separate Elektrodenkontaktflächen an dem Elektrodenkopf (2) ausgebildet sind, wobei die separaten Elektrodenkontaktflächen (22a, 22b) mit separaten Signalleitungen in dem flexiblen Kabel (3; 3') verbunden sind.

10. Gastrointestinalelektrode nach Anspruch 9, wobei die separaten Elektrodenkontaktflächen (22a, 22b) als separate Umfangsringe um den Elektrodenkopf (2) herum ausgebildet sind.

## Revendications

1. Dispositif d'électrode gastro-intestinale (1) comprenant une tête d'électrode (2) destinée à être implantée dans une paroi d'estomac pour l'émission ou la réception de signaux électriques et un câble flexible (3 ; 3') avec une couche d'isolation extérieure (32), ledit câble flexible (3 ; 3') étant relié à ladite tête d'électrode (2) pour transférer les signaux électriques à un côté externe dudit dispositif d'électrode (1) ;
dans lequel ladite tête d'électrode (2) comprend un moyen de rétention extensible flexible et au moins une zone de contact d'électrode (22) agencée pour couvrir au moins une partie de la zone de tête d'électrode extérieure ; et
dans lequel le moyen de rétention extensible est automatiquement extensible d'un état d'insertion à un état étendu pour s'opposer à la rétraction de l'électrode à travers l'ouverture d'insertion, en rendant de ce fait l'électrode attachable à la paroi d'estomac ;
dans lequel la tête d'électrode (2) comprend en outre une zone de butée (23 ; 23') et le dispositif d'électrode comprend en outre une partie d'introduction relativement rigide (4 ; 4'), agencée pour se mettre en prise avec ladite zone de butée (23 ; 23') de la tête d'électrode (2) pour une introduction percutanée du cathéter dans la paroi d'estomac à travers une ouverture, dans lequel la partie d'introduction (4 ; 4') est libérable de la tête d'électrode (2) après insertion, de telle manière que la tête d'électrode (2) reste implantée dans la paroi d'estomac ; et
**caractérisé en ce que** le moyen de rétention extensible comprend au moins une aile de rétention flexible (21) ;
dans lequel la tête d'électrode est dimensionnée pour être entièrement contenue à l'intérieur de la paroi d'estomac.

2. Electrode gastro-intestinale selon la revendication 1, dans laquelle chacune de l'au moins une aile de rétention flexible (21) a une largeur inférieure ou égale au diamètre de la tête d'électrode (2).

3. Electrode gastro-intestinale selon la revendication 2, dans laquelle le moyen de rétention extensible comprend au moins deux ailes de rétention flexibles (21), agencées de préférence en étant séparées de manière équidistante dans une direction circonférentielle autour de l'électrode.

4. Electrode gastro-intestinale selon l'une quelconque des revendications précédentes, dans laquelle la tête d'électrode (2) comprend en outre une portion d'embout arrondie (24), ladite portion d'embout arrondie ne faisant pas partie de la zone de contact d'électrode (22).

5. Electrode gastro-intestinale selon l'une quelconque des revendications précédentes, dans laquelle la zone de contact électrode (22) est agencée entre le moyen de rétention extensible flexible (21) et une liaison entre le câble flexible (3) et la tête d'électrode (2).

6. Electrode gastro-intestinale selon l'une quelconque des revendications précédentes, dans laquelle la partie d'introduction (4) comprend une partie tubulaire agencée pour être disposée à l'extérieur du câble flexible (3), dans laquelle ladite zone de butée (23) est formée en tant que zone d'épaulement faisant saillie sur ladite tête d'électrode (2).

7. Electrode gastro-intestinale selon l'une quelconque des revendications 1 à 5, dans laquelle la partie d'introduction (4') comprend une tige épaisse agencée pour être disposée à l'intérieur du câble flexible (3'), dans laquelle ladite zone de butée (23') est formée en tant que butée d'évidement ou de protubérance centrale dans la tête d'électrode (2).

8. Electrode gastro-intestinale selon l'une quelconque des revendications précédentes, destinée à être utilisée pour la commande et/ou l'observation de la fonction motrice dans l'estomac.

9. Electrode gastro-intestinale selon l'une quelconque des revendications précédentes, dans laquelle deux zones de contact d'électrode distinctes sont fournies sur ladite tête d'électrode (2), dans laquelle lesdites zones de contact d'électrode distinctes (22a, 22b) sont reliées à des lignes de signal distinctes dans ledit câble flexible (3 ; 3').

10. Electrode gastro-intestinale selon la revendication 9, dans laquelle les zones de contact d'électrode distinctes (22a, 22b) sont fournies en tant qu'anneaux circonférentiels distincts autour de la tête d'électrode (2).
